Europäisches Patentamt

European Patent Office

Office européen des brevets

(19)

(11) Veröffentlichungsnummer: **0 262 310**
**A1**

## (12) EUROPÄISCHE PATENTANMELDUNG

(21) Anmeldenummer: 87109437.1

(22) Anmeldetag: 01.07.87

(51) Int. Cl.⁴: **C07B 35/08** , C07C 33/025 , C07C 49/203 , C07C 57/03 , C07C 69/52 , C07C 29/56 , C07C 45/67 , C07C 51/353 , C07C 67/333

(30) Priorität: 29.08.86 DE 3629512

(43) Veröffentlichungstag der Anmeldung:
06.04.88 Patentblatt 88/14

(84) Benannte Vertragsstaaten:
BE CH DE FR GB IT LI NL

(71) Anmelder: HÜLS AKTIENGESELLSCHAFT
Patentabteilung / PB 15 - Postfach 13 20
D-4370 Marl 1(DE)

(72) Erfinder: Bickert, Peter, Dr.
318 Westgate Dr.
North Edison New Jersey 08820(US)
Erfinder: Kaufhold, Manfred, Dr.
Jasminweg 20
D-4370 Marl(DE)

(54) **Verfahren zur Isomerisierung unverzweigter funktionalisierter Verbindungen mit endständigen Doppelbindungen.**

(57) Zur Isomerisierung unverzweigter funktionalisierter Alkene mit terminaler Kohlenstoff-Kohlenstoff-Doppelbindung unter Erhalt des Kohlenstoffgerüstes behandelt man diese in Gegenwart von Rhodium(III)- oder Iridium(III)verbindungen bei Temperaturen von 70 bis 160 °C. Als funktionalisierte Verbindungen mit endständiger Doppelbindung setzt man Alpha-Omega-Alkenole, deren Ester, Alpha-Omega-Alkencarbonsäuren und deren Ester ein, als Rhodium(III)verbindungen vorzugsweise Rhodium(III)halogenide, insbesondere Rhodium(III)chloridhydrat. Die Reaktionsprodukte enthalten die Kohlenstoff-Kohlenstoff-Doppelbindung nicht in Konjugation zur funktionellen Gruppe.

EP 0 262 310 A1

## Verfahren zur Isomerisierung unverzweigter funktionalisierter Verbindungen mit endständigen Doppelbindungen

Die Erfindung betrifft ein Verfahren zur Isomerisierung unverzweigter, funktionelle Gruppen tragender Alkene mit einer oder zwei endständigen Kohlenstoff-Kohlenstoff-Doppelbindungen durch Verschiebung derselben unter Erhalt des Kohlenstoff-Gerüstes durch Einwirkung von einfachen oder komplexen Salzen des Rhodiums oder Iridiums.

Ungesättigten organischen Verbindungen mit unverzweigter Kohlenstoffkette kommt als Riechstoffen eine erhebliche Bedeutung zu. Dabei ist der Geruch sowohl einzelner solcher Verbindungen als auch daraus erhaltener Gemische in charakteristischer Weise von der genauen chemischen Struktur und damit auch von der Lage der Kohlenstoff-Kohlenstoff-Doppelbindungen im Riechstoff-Molekül abhängig. Zur Einstellung von Geruchs-Nuancen ist es somit wünschenswert, über eine Palette ähnlicher, sich in der jeweiligen Stellung der Doppelbindung unterscheidender Verbindungen und entsprechender Gemische zu verfügen. Als Ausgangsmaterialien zur Herstellung derartiger Produkte stehen technisch insbesondere solche Verbinbungen zur Verfügung, in welchen sich die Kohlenstoff-Kohlenstoff-Doppelbindung am Ende der Kohlenstoffkette des Moleküls befindet (terminale Position). Dazu zählen beispielsweise 10-Undecensäure (Undecylensäure) sowie deren Abkömmlinge wie 10-Undecensäurealkylester, 10-Undecenol und dessen Ester, aber auch vergleichbare Verbindungen mit Kohlenstoffketten anderer Länge, beispielsweise 7-Octensäure und deren Derivate (EP-OS 0 069 339).

Zur Isomerisierung organischer Verbindungen durch Verschiebung der Kohlenstoff-Kohlenstoff-Doppelbindungen existieren bereits seit langer Zeit zahlreiche Methoden von teilweise erheblicher wirtschaftlicher Bedeutung. Die hierbei vornehmlich angewandten Verfahren bedienen sich jedoch entweder der heterogenen Katalyse, hoher Temperatur und Drucke oder aber stark alkalischer Reaktionsmedien. Daher sind solche Verfahren üblicherweise nicht zur Umsetzung empfindlicher, funktionelle Gruppen tragender Alkene geeignet.

In neuerer Zeit sind jedoch auch Verfahren zur Isomerisierung von Alkenen mittels homogener Katalyse durch Übergangsmetallkomplexe entwickelt worden. Neben Verbindungen des Nickels und des Palladiums sind hierzu insbesondere Rhodium-Verbindungen untersucht worden, von welchen vor allem das Tris(triphenylphosphin)-rhodium(I)chlorid (Wilkinson-Katalysator) eine gewisse Bedeutung erlangt hat. Auch andere Rhodium(I)-Komplexe sowie Verbindungen des dreiwertigen Rhodiums und entsprechende Iridiumverbindungen finden als Isomerisierungs-Katalysatoren Verwendung (R. S. Dickson, Homogeneons Catalysers with Compounds of Rhodium and Iridium, Dordrecht 1985, S. 14 bis 18).

Als Substrate bekannter Isomerisierungsreaktionen wurden vornehmlich Kohlenwasserstoffe eingesetzt. Daneben sind jedoch einige Beispiele bekannt, in welchen funktionalisierte Alkene eine Verschiebung der Kohlenstoff-Kohlenstof-Doppelbindungen erfuhren, beispielsweise die Umwandlung von But-3-ensäure in But-2-ensäure (Crotonsäure) durch Tris(triphenylphosphin)-rhodium(I)-chlorid/Zinn(II)chlorid (DE-OS 20 49 937), von Itaconsäuredimethylester in Mesaconsäuredimethylester und Citraconsäuredimethylester durch den gleichen Katalysator (H. Singer et al., Tetrahedron 28, 5769 (1972); M. Sakai et al., J. Chem. Soc. Jpn. 1981, 1283), von Alpha-Beta-ungesättigten Ketonen in andere mit höherer Substituentenzahl an der Kohlenstoff-Kohlenstoff-Doppelbindungen durch Rhodium(III)chlorid (P. Grieco et al., Tetrahedron Lett. 1978, 2545; P. Grieco et al., J. Am. Chem. Soc. 98, 7102 (1976)), sowie von N-Alkylamiden und -imiden zu Enamiden durch den Wilkinson-Katalysator oder Rhodium(III)chlorid (J. K. Stille et al., J. org. Chem. 45, 2139 (1980)).

Auch die Isomerisierung von Penten-4-säuremethylester mit dem Katalysatorsystem Tris(triphenylphosphin)-rhodium(I)chlorid/Zinn(II)-chlorid wurde untersucht, wobei gezeigt werden konnte, daß stets der konjugierte Alpha-Beta-ungesättigte Carbonsäureester bevorzugt gebildet wird.

Zum Stand der Technik gehören demnach Verfahren zur Isomerisierung nicht funktionalisierter und funktionalisierter Alkene, auch solchen mit terminaler Kohlenstoff-Kohlenstoff-Doppelbindung, durch in homogener Phase katalytisch wirksame Übergangsmetallverbindungen unter Bildung von Produkten bzw. Produktgemischen, welche als Hauptbestandteil dasjenige Isomere enthalten, in welchen die Kohlenstoff-Kohlenstoff-Doppelbindung in Konjugation zur funktionellen Gruppe steht.

Von den gewünschten Produkten war jedoch zu fordern, daß sie die Kohlenstoff-Kohlenstoff-Doppelbindung weder am Ende des Moleküls noch in einer zur funktionellen Gruppen konjugierten Stellung aufweisen. Vertreter dieser Verbindungsklasse sind bekannt, beispielsweise 2-Undecensäure (English and Gregory, J. Am. Chem. Soc. 69 (1947) 2121), 2-Undecensäurebutylester (Colland, Helv. Chim. Acfa 26 (1943) 1157) oder 2-Undecenol (Swift et al., J. Am. Chem. Soc. 71 (1949) 1512) und können u. a. kernresonanzspeletroskopisch leicht nachgewiesen werden (R. R. Fraser und D. E. McGreer, Can. J. Chem. 39 (1961) 505; D. H. Williams und I. Fleming, Spektroskopische Methoden zur Strukturaufklärung, Stuttgart, 1975).

Hieraus ergab sich die Aufgabe, ein einfaches und wirtschaftliches Verfahren zur finden, nach welchem funktionalisierte Alkene mit innenständiger, jedoch nicht in Konjugation zu einer funktionellen Gruppe stehenden Doppelbindung durch Isomerisierung geeigneter Edukte mit terminaler Doppelbindung erhalten werden können.

Diese Aufgabe wird erfindungsgemäß entsprechend den Angaben der Patentansprüche gelöst.

Dem Stand der Technik entsprechend war davon auszugehen, daß von den möglichen Isomerisierungsprodukten dasjenige mit konjugierter Doppelbindung das thermodynamisch stabilste ist und ihm demzufolge eine entsprechend hohe Bildungstendenz zukommt. Überraschend wurde jedoch gefunden, daß bei der Isomerisierung unverzweigter funktionalisierter Alkene mit terminaler Kohlenstoff-Kohlenstoff-Doppelbindung, welche vorzugsweise durch wenigstens 5, insbesondere 7 bis 9 Kohlenstoff-Atome vom funktionalisierten Kohlenstoff-Atom entfernt ist, durch Rhodium(III)-verbindungen, insbesondere Rhodium(III)-chloridhydrat (RhCl$_3$ • 3 H$_2$O) Produkte erhalten werden, in welchen die Kohlenstoff-Kohlenstoff-Doppelbindung nicht in Konjugation zur funktionellen Gruppe steht. Selbst bei vergleichsweise langen Reaktionszeiten werden keine Anzeichen für die Bildung von Produkten gefunden, in denen die Doppelbindung in Konjugation zur funktionellen Gruppen steht.

Zur Durchführung des erfindungsgemäßen Verfahrens setzt man vorzugsweise Rhodium(III)-halogenide wie Rhodium(III)chlorid, Rhodium(III)-bromid, Rhodium(III)iodid und/oder andere Rhodium(III)salze wie Rhodium(III)nitrat, Rhodium-(III)sulfat oder deren Hydrate, insbesondere Rhodium(III)chloridhydrat (RhCl$_3$ • 3 H$_2$O) als Katalysatoren ein. Auch andere Rhodium(III)-Komplexe, beispielsweise Tris(triphenylphosphin)rhodium(III)-chlorid, Tris(benzonitril)rhodium(III)chlorid, Tris-(pyridin)rhodium(III)chlorid und Rhodium(III)-acetylacetonat sowie die den Rhodium(III)-Komplexen analogen Iridium(III)verbindungen sind geeignet.

Zur Isomerisierung wird der Katalysator homogen gelöst. Man setzt ihn in einer Konzentration von 0.01 bis 10.0 g/kg Reaktionslösung, bevorzugt 0.1 bis 1 g/kg Reaktionslösung, ein.

Ein besonderer Vorteil des erfindungsgemäßen Verfahrens ist es, daß die eingesetzten Katalysatoren Derivate jeweils eines einzigen Metalls (Rhodium oder Iridium) sind und es nicht erforderlich ist, einen aktivitätssteigernden Katalysator zuzusetzen, wie dies beispielsweise für Tris-(triphenylphosphin)rhodium(I)-chlorid mit Zinn(II)-chlorid der Fall ist (DE-OS 20 49 937; H. Singer et al., Tetrahedron 28 (1972) 5769). Auf diese Weise ist es möglich, das Verfahren zur Rückgewinnung des Edelmetalls wesentlich zu vereinfachen, da die Abtrennung eines anderen Metalls nicht erforderlich ist.

Überraschend wurde darüber hinaus gefunden, daß bei der Verwendung von Rhodium(III)salzen die Isomerisierung terminaler funktionalisierter Alkene keine ausgedehnte Induktionsperiode geringer Katalysator-Akti vität zu Beginn der Reaktion auftritt, wie dies vom Katalysatorsystem Rhodium(III)-chlorid/Zinn(II)chlorid bekannt ist (H. Singer et al., Tetrahedron 28, 5769 (1972)).

Die zur Isomerisierung nach dem erfindungsgemäßen Verfahren geeigneten funktionalisierten Verbindungen mit endständiger Doppelbindung tragen wenigstens einen Heterosubstituenten am Kohlenstoff-Gerüst, beispielsweise Hydroxyl-, Keto-, Carboxyl-Funktionen oder Carbonsäureester-Gruppen oder auch deren Kombinationen.

Nicht geeignet sind jedoch Aldehyde, da diese den Katalysator desaktivieren. Bevorzugt setzt man Alpha-Omega-Alkenole, deren Ester, Alpha-Omega-Alken-Carbonsäuren oder Alpha-Omega-Alken-Carbonsäureester oder Di(Omega-alkenyl)Ketone mit unverzweigter Alkenkohlenkette ein. Zwischen der terminalen Kohlenstoff-Kohlenstoff-Doppelbindung und dem eine funktionelle Gruppe tragenden Kohlenstoff befinden sich 5 bis 15, bevorzugt 6 bis 10, insbesondere 7 oder 8, Kohlenstoffatome.

Das erfindungsgemäße Verfahren gestattet die Isomerisierung funktionalisierter terminaler Alkene in Gegenwart und in Abwesenheit von Lösemitteln. Geeignete Lösemittel für die homogen-katalysierte Isomerisierung sind Alkohole, Ether, Ketone, Ester, Amide, Nitrile, Carbonsäuren, Wasser oder deren Gemische. Mit diesen Zusätzen kann man während der Isomerisierung die funktionellen Gruppen auch abwandeln. Beispielsweise kann eine ungesättigte Carbonsäure mit einem Alkohol zum entsprechenden Ester verestert werden oder ein ungesättigter Alkohol kann mit einer Carbonsäure während der Isomerisierung verestert werden. Beide Reaktionen, Isomerisierung und Veresterung laufen somit gleichzeitig ab.

Nach der Isomerisierung kann der Katalysator beispielsweise durch Extraktion mit verdünnten Säuren, vorzugsweise jedoch durch Destillation der Reaktionsprodukte aus dem Reaktionsgefäß abgetrennt werden. Die destillative Abtrennung hat den Vorteil, daß solchermaßen zurückgewonnener Katalysator direkt für weitere Isomerisierungen eingesetzt werden kann. Bei absatzweitem Betrieb der Isomerisierung hat es sich überraschend als günstig erwiesen, nur etwa bis zu 80 % des eingesetzten Reaktionsgemisches abzudestillieren und dieses dann durch eine entsprechende Menge Ausgangsmaterial zu ersetzen. Dadurch gelingt es, die im Prozeßverlauf auftretende Desaktivierung des Katalysators einzuschränken. Es ist somit ohne weiteres möglich, einen einmal zur Isomerisierung eingesetzten Katalysator für mehrere Reaktionsansätze hintereinander zu verwenden.

Die ursprüngliche Aktivität läßt sich dabei in einfacher Weise wieder zurückgewinnen, indem man eine dem desaktivierten Anteil entsprechenden Menge, gewöhnlich 10 bis 80 %, vorzugsweise 25 bis 60 % des ursprünglich eingesetzten Katalysators, erneut zusetzt. Ein besonderer Vorteil dieser Verfahrensweise ergibt sich aus der dadurch erzielten Anreicherung von Katalysator-Rückständen aus mehreren Folgeansätzen im Sumpf eines Reaktionsgefäßes und der dadurch möglichen wirtschaftlicheren Rückgewinnung des eingesetzten Edelmetalls.

Die Isomerisierung führt man in einem Temperaturbereich von 70 bis 160 °C durch, bevorzugt bei 100 bis 130 °C. Sie kann absatzweise oder auch im kontinuierlichen Betrieb erfolgen. In Abhängigkeit von der Konzentration des eingesetzten Katalysators und der Reaktionstemperatur ist für einen Umsatz > 95 % im allgemeinen eine Reaktionszeit von 1 bis 24 Stunden erforderlich. Die Selektivität liegt auch bei hohen Umsätzen bei > 95 %.

Die Anwesenheit von Basen wie Alkali oder Aminen im Reaktionsgemisch führt zur Verminderung der Reaktionsgeschwindigkeit und zum Aktivitätsverlust des Katalysators. Durch Zugabe geringer Mengen einer starken Säure, beispielsweise einer Mineralsäure wie Schwefelsäure, Salzsäure oder Phosphorsäure kann man die Wirkung der Basen kompensieren und die Isomerisierung beschleunigen.

Im allgemeinen setzt man die Säure in Mengen von 0.1 bis 5 %, bezogen auf die Reaktionslösung zu.

Die Isomerisierung kann man auch unter einem Inertgas, vorzugsweise Stickstoff, durchführen. Überraschenderweise kann die erfindungsgemäße Isomerisierung jedoch auch in Gegenwart von Luft ausgeführt werden, obwohl bekannt ist, daß Sauerstoff terminale Alkene in Gegenwart von Rhodium-(III)-Salzen, insbesondere Rhodium(III)chloridhydrat, oxidiert und in Ketone überführt (R. S. Drago et al., J. Am. Chem. Soc. $\underline{107}$, 2898 (1985)).

Beispiel 1

48 g (0.2 Mol) 10-Undecensäurebutylester wurden mit einer Lösung von 53 mg RhCl$_3$ • 3 H$_2$O in 50 ml n-Butanol und 2 g konz. Schwefelsäure versetzt und auf 120 °C erhitzt. Der Reaktionsverlauf wurde stündlich durch gaschromatographische Untersuchung entnommener Proben verfolgt. Nach 5 h war die Zusammensetzung des Reaktionsgemisches praktisch konstant. Man ließ abkühlen, setzte 500 ml Diethylester zu und wusch mit verdünnter Essigsäure und Wasser. Die organische Phase wurde über Calciumchlorid getrocknet, eingeengt und destilliert. Bei 89 bis 92 °C/0.05 mbar erhielt man 37.7 g (79 %) eines Gemisches isomerer Undecensäurebutylester. Der Anteil an 10-Undecensäurebutylester wurde protonenresonanzspektroskopisch zu < 5 % bestimmt. 2-Undecensäurebutylester wurde nicht nachgewiesen.

Beispiel 2

480 g (2 Mol) 10-Undecensäurebutylester wurden mit einer Lösung von 53 mg RhCl$_3$ • 3 H$_2$O in 50 ml n-Butanol versetzt und 24 h auf 120 °C erhitzt. Danach arbeitete man destillativ auf und erhielt bei 108 bis 113 °C/0.4 bis 0.6 mbar eine Hauptfraktion von 444 g (93 %) isomerer Undecensäurebutylester mit einem protonenresonanzspektroskopisch bestimmten Anteil an 10-Undecensäurebutylester von < 5 %. Der bei der Destillation aus dem Sumpf gewonnene Rückstand (6 g) enthielt den Katalysator und wurde ohne besondere Vorkehrungen 18 Tage an der Luft bei Raumtemperatur aufbewahrt. Danach wurden erneut 480 g (2 Mol) 10-Undecensäurebutylester und 50 ml n-Butanol zugesetzt und das erhaltene Gemisch 20 h auf 120 °C erhitzt. Die anschließende Destillation ergab bei 94 bis 104 °C/0.2 mbar eine Hauptfraktion von 465 g (97 %), deren Zusammensetzung protonenresonanzspektroskopisch zu 53 % isomerisierter Undecensäurebutylester und 47 % 10-Undecensäurebutylester ermittelt wurde.

Beispiel 3

Zu 12.0 kg (50 Mol) 10-Undecensäurebutylester setzte man eine Lösung von 1.3 g RhCl$_3$ • 3 H$_2$O in 1.25 l n-Butanol und erhitzte 24 h auf 120 °C. Durch Destillation gewann man anschließend bei 119 bis 126 °C/2 mbar 11.3 kg (94 %) isomerisierten Undecensäurebutylester (< 5 % 10-Undecensäurebutylester gem. $^1$H-NMR). Den Rückstand versetzte man erneut mit 12.0 kg (50 Mol) 10-Undecensäurebutylester und einer Lösung von 0.5 g RhCl₃ • 3 H₂O in 1.25 l n-Butanol. Nach 24 h bei 120 °C ergab die destillative Aufarbeitung wie oben 11.7 kg (98 %) isomerisierten Undecensäurebutylester (Gehalt an 10-Undecensäurebutylester gem. ¹H-NMR: 7 %).


Beispiel 4

36.8 g (0.2 Mol) 10-Undecensäure wurden mit einer Lösung von 53 mg RhCl₃ • 3 H₂O in 1 ml Wasser versetzt und auf 100 °C erwärmt. Auf eine Rückführung des aus dem Reaktionsraum entweichenden Wassers wurde verzichtet. Nach 6 h gewann man durch Destillation bei 105 bis 107 °C/0.07 mbar 29.8 g (81 %) eines Gemisches isomerer Undecensäuren. Protonenresonanzspektroskopisch wurde der Gehalt an 10-Undecensäure zu < 10 % bestimmt. 2-Undecensäure wurde nicht nachgewiesen.


Beispiel 5

Zu 73.6 g (0.4 Mol) 10-Undecensäure gab man eine Lösung von 106 mg RhCl₃ • 3 H₂O in 100 ml n-Butanol und erhitzte 6 h auf 100 °C. Danach zog man überschüssiges n-Butanol ab und destillierte den Rückstand im Vakuum. Bei 98 bis 100 °C/0.06 mbar erhielt man 77 g (80 %) eines Gemisches isomerer Undecensäurebutylester mit < 4 % 10-Undecensäurebutylester.


Beispiel 6

Zu 17.0 g (0.1 Mol) 10-Undecenol gab man eine Lösung von 26 mg RhCl₃ • 3 H₂O in 30 ml n-Butanol und erhitzte 4 h zum Rückfluß. Nach Abziehen des n-Butanols erhielt man bei 77 bis 81 °C/0.02 mbar 14.9 g (88 %) isomerisierter Undecenole, deren Gehalt an 10-Undecenol protonenresonanzspektroskopisch zu < 4 % bestimmt wurde.


Beispiel 7

Eine Lösung von 53 mg RhCl₃ • 3 H₂O in 60 mol Eisessig wurde mit 34.0 g (0.2 Mol) 10-Undecenol versetzt und 6 h auf 100 °C erhitzt. Durch destillative Aufarbeitung erhielt man bei 70 bis 75 °C/0.2 mbar 30.2 g (89 %) ein Gemisch isomerer Undecenylacetate mit < 4 % 10-Undecenylacetat (gem. ¹H-NMR).

Beispiel 8

Eine Lösung aus 30.6 g (0.1 Mol) 1,20-Heneicosadien-11-on (Schmp. 50 bis 52 °C) und 53 mg RhCl₃ • 3 H₂O in 60 ml n-Butanol wurde 6 h auf 100 °C erhitzt. Danach destillierte man das n-Butanol ab, nahm den Rückstand in Ether auf und extrahierte mit verdünnter Essigsäure und Wasser. Nach dem Trocknen der organischen Phase mit Natriumsulfat zog man den Ether ab und erhielt so 27.0 g (88 %) eines Gemisches isomerer Heneicosadien-11-one (Schmp. 43 bis 46 °C).


Charakterisierung der Produkte:

Zur eindeutigen Charakterisierung der beschriebenen Verbindungen werden je ein Protonenresonanzspektrum und ein Gaschromatogramm folgender Stoffe abgebildet:

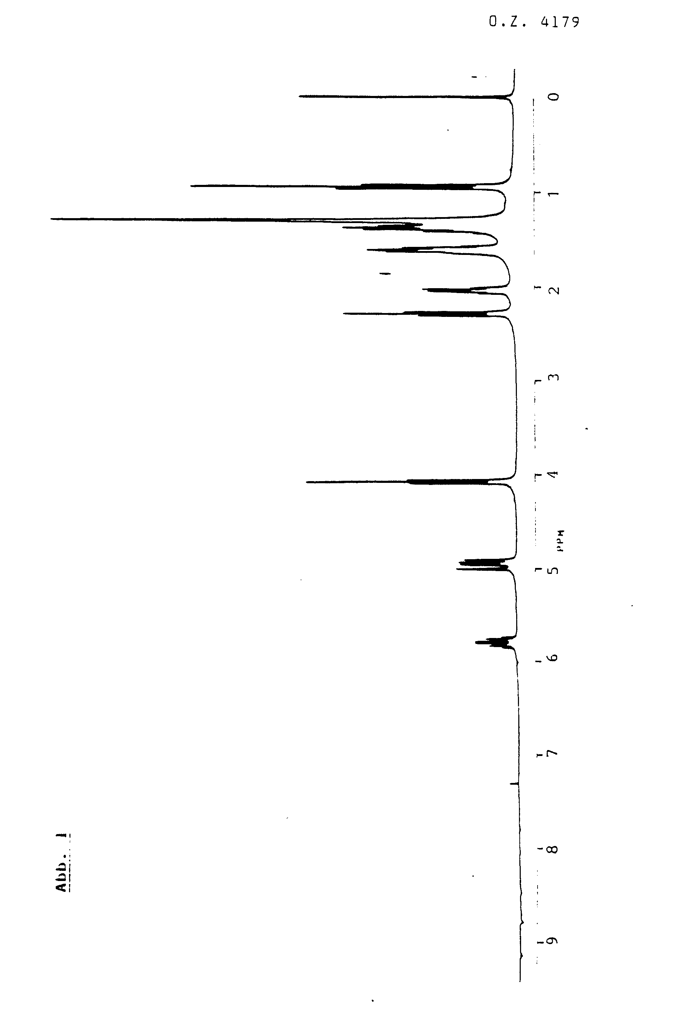

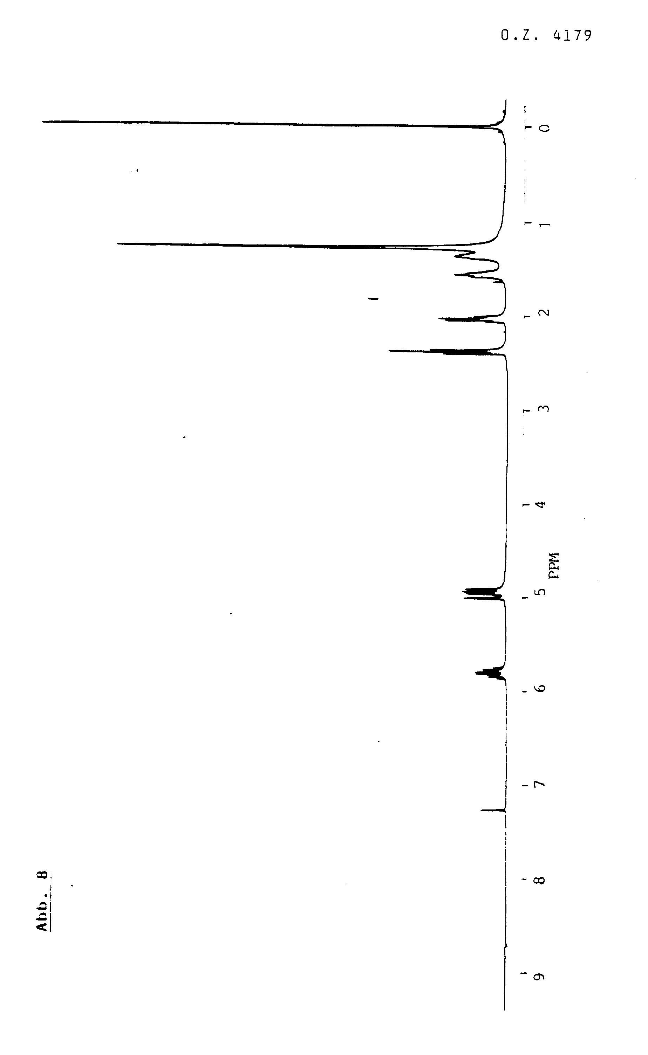

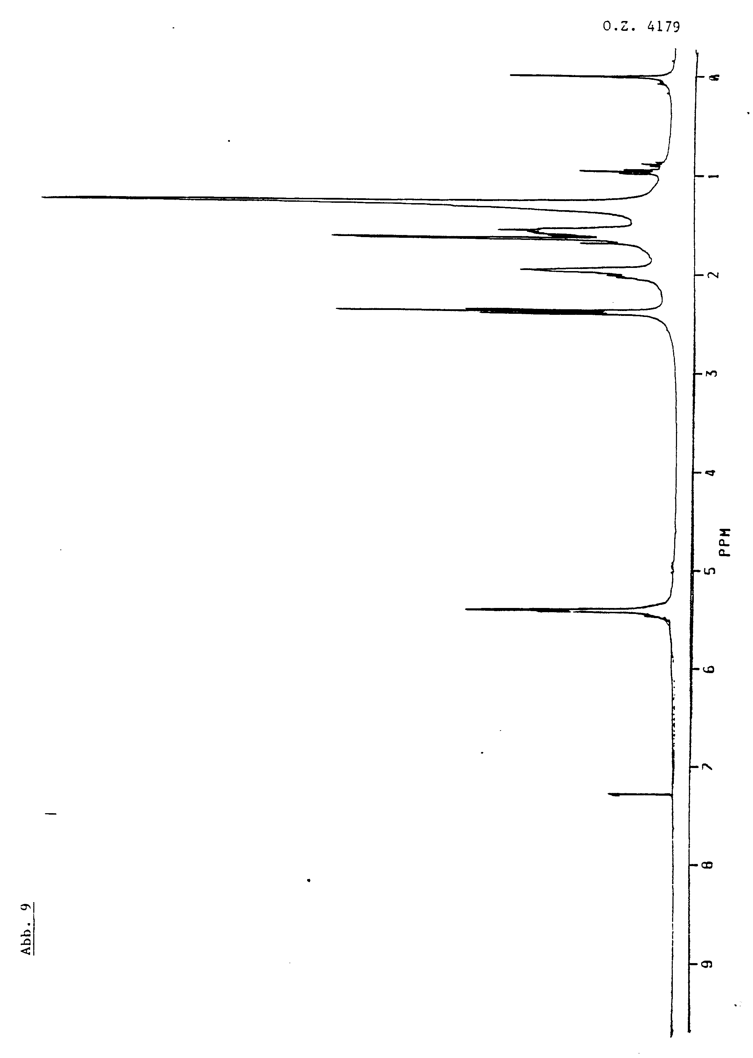

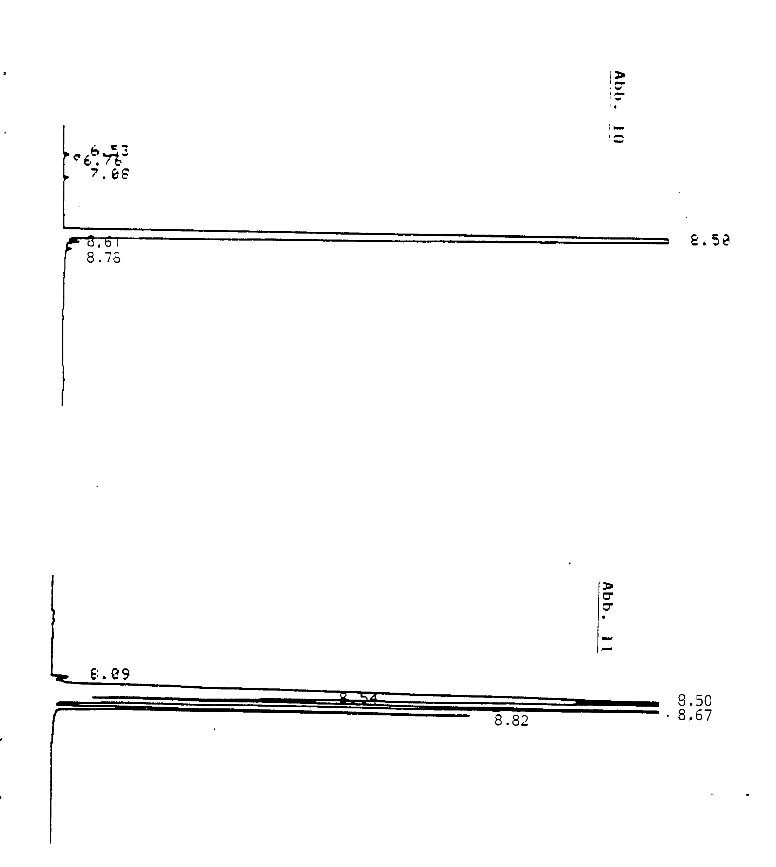

    1. 10-Undecensäurebutylester     Abb. 1 + 10

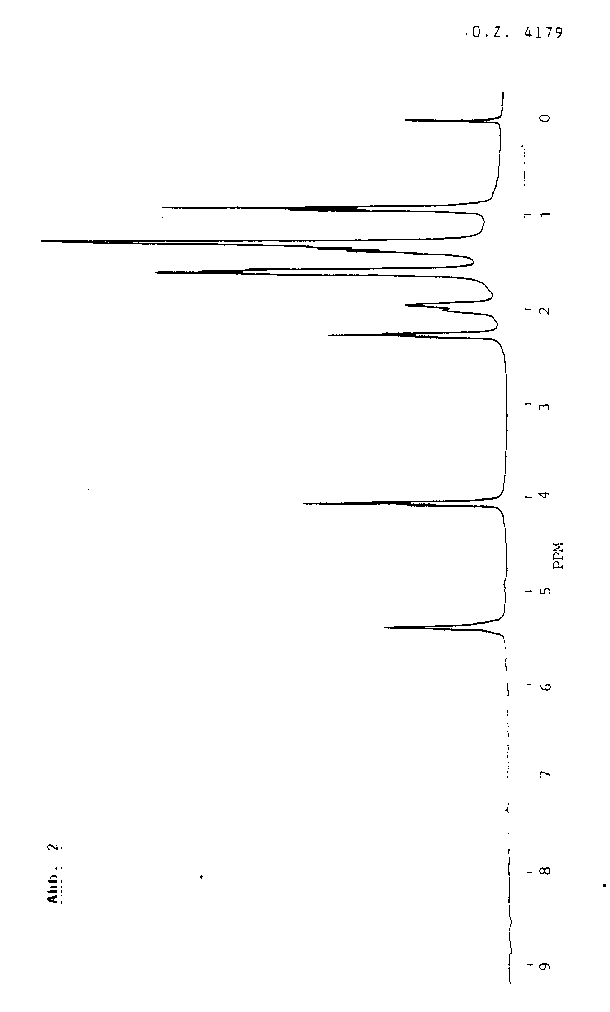

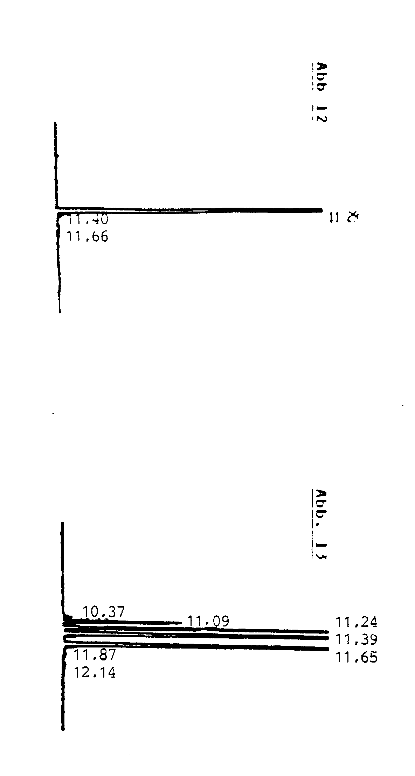

    2. Gemisch isomerer Undecensäurebutylester     Abb. 2 + 11

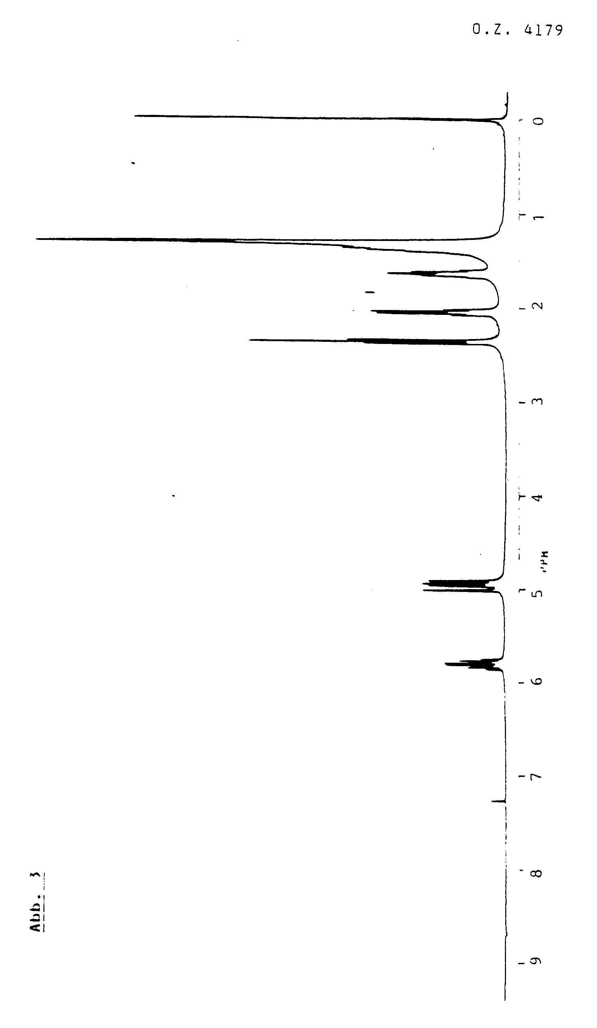

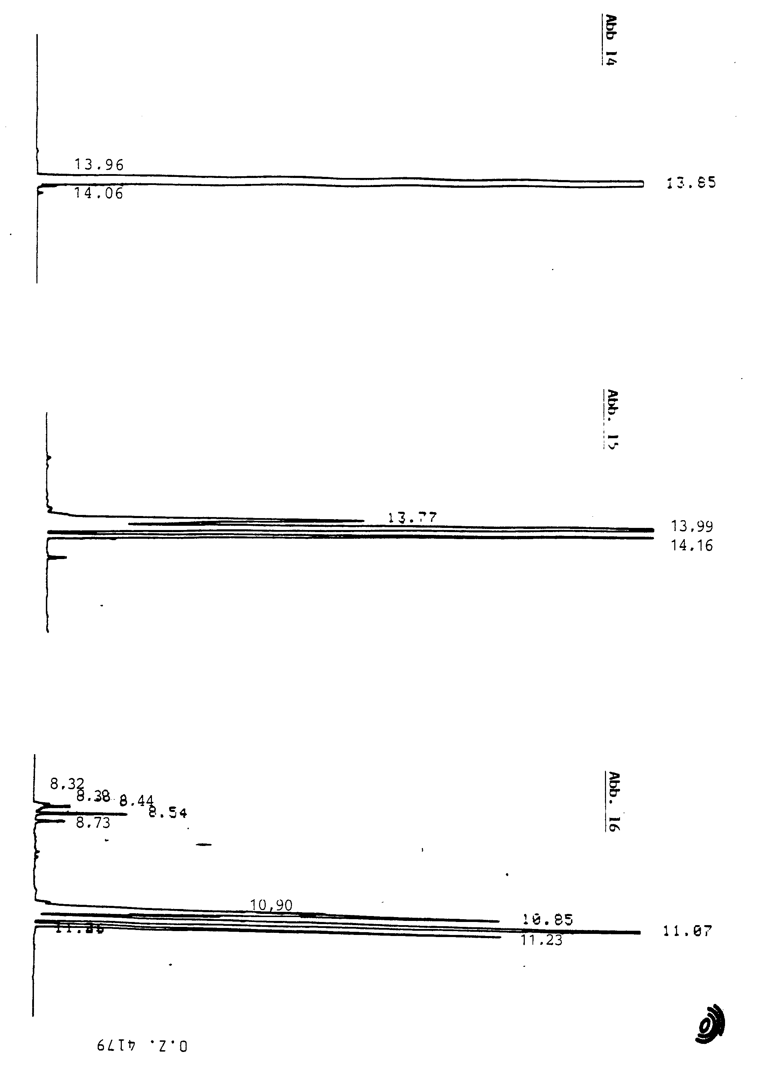

    3. 10-Undecensäure     Abb. 3 + 12

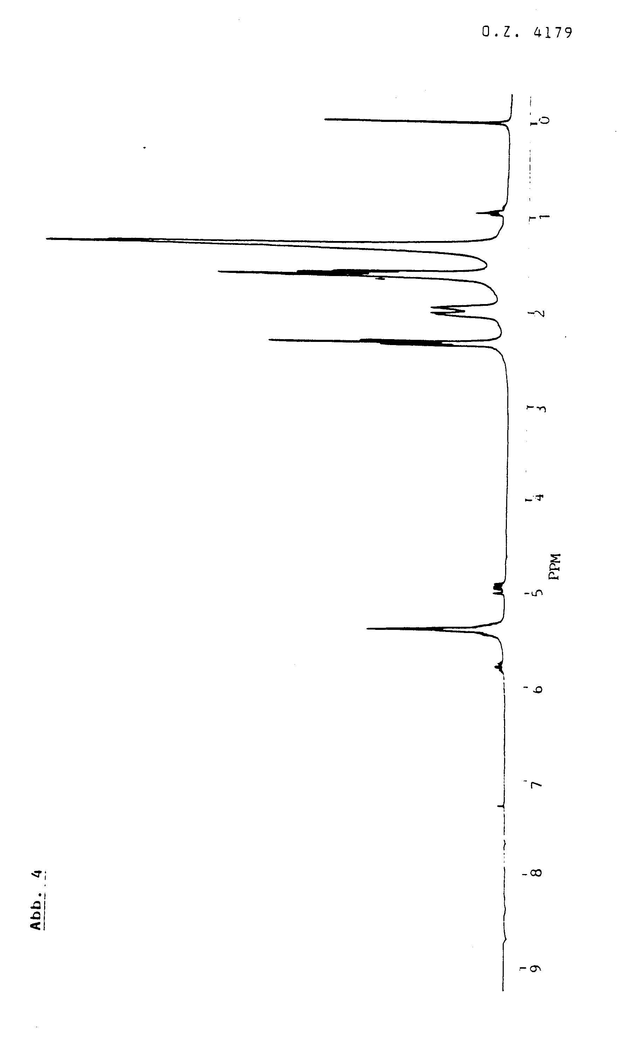

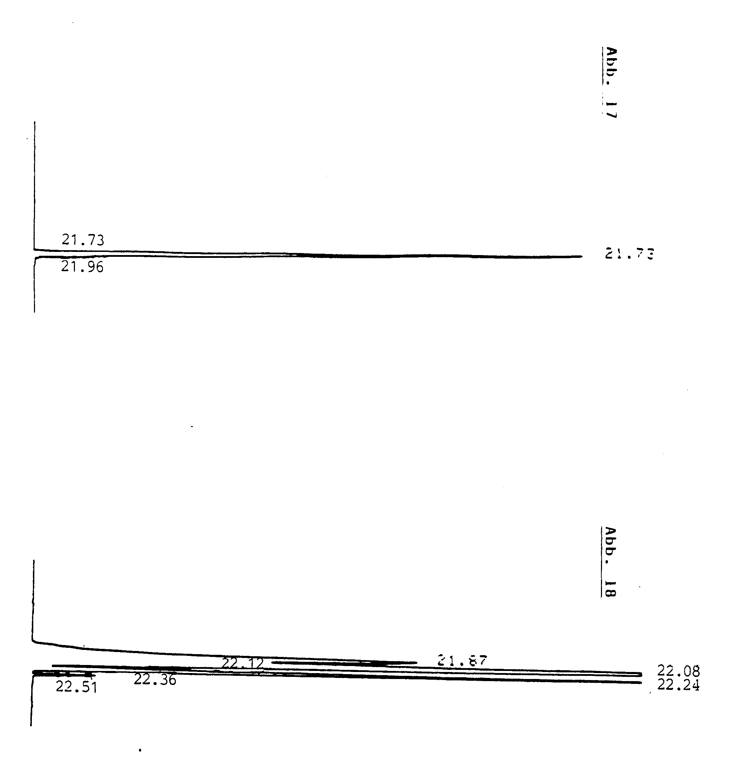

    4. Gemisch isomerer Undecensäuren Abb. 4 + 13

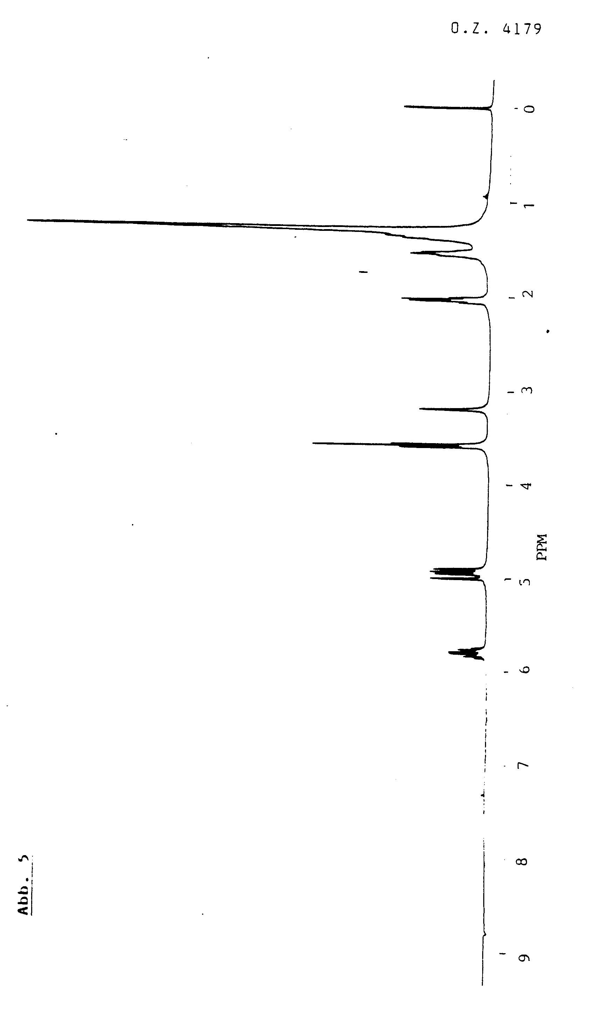

    5. 10-Undecenol     Abb. 5 + 14

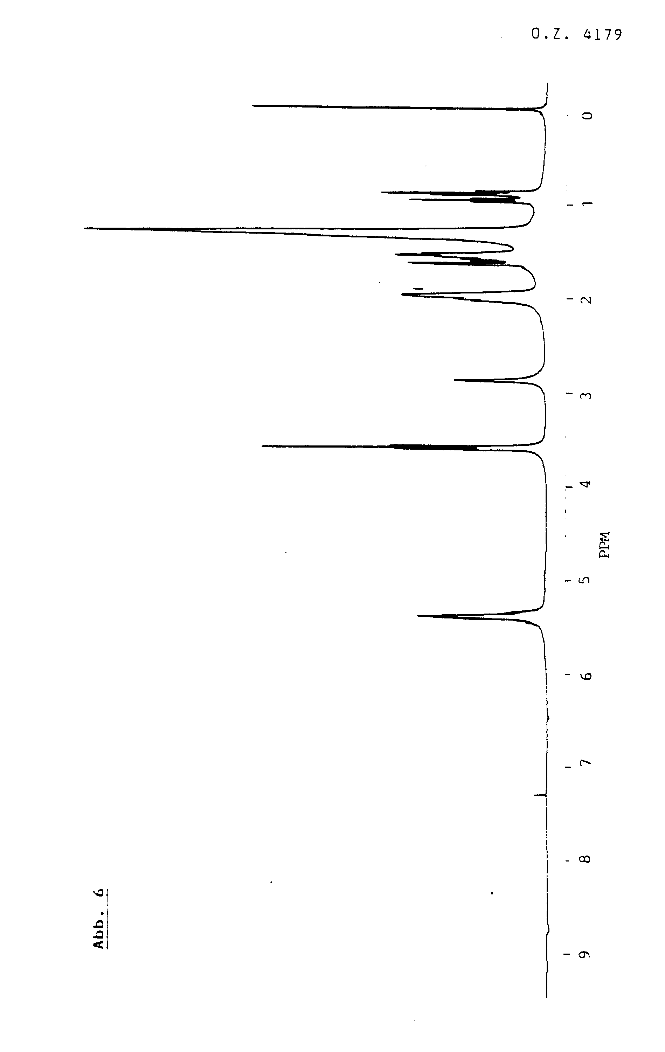

    6. Gemisch isomerer Undecenole     Abb. 6 + 15

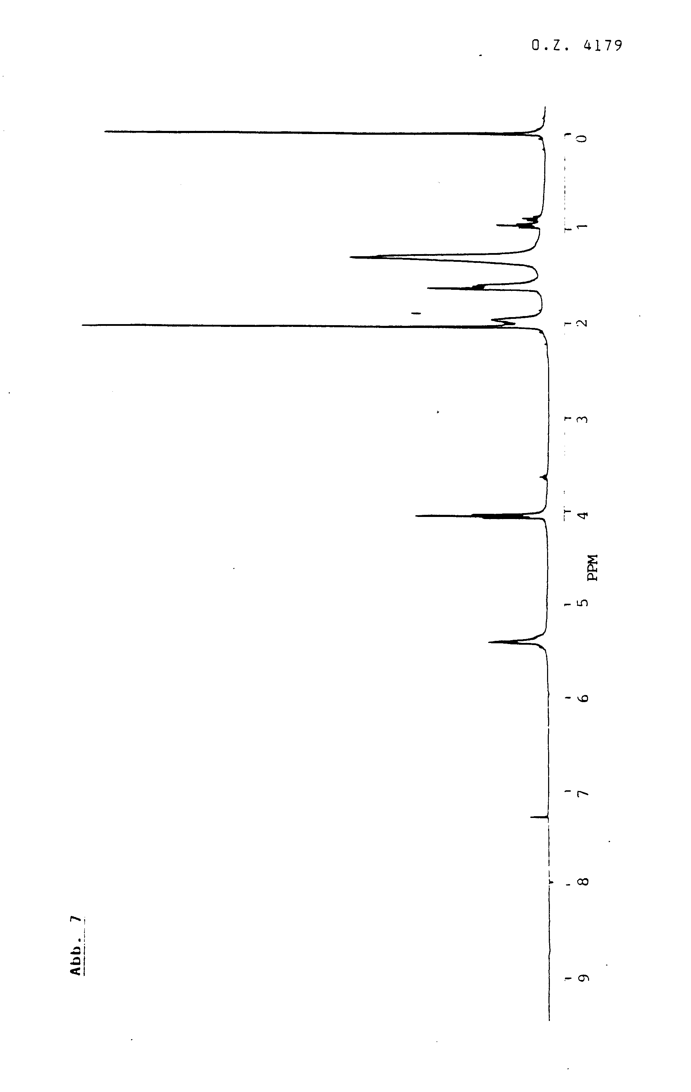

    7. Gemisch isomerer Undecylacetate Abb. 7 + 16

    8. 1,20-Heneicosadien-11-on     Abb. 8 + 17

    9. Gemisch isomerer Heneicosadien-11-one Abb. 9 + 18

    (¹H-NMR in CDCl₃, Delta-Skala; GC auf 25 m Sil 05, 100 bis 300 °C)


**Ansprüche**

1. Verfahren zur Isomerisierung unverzweigter funktionalisierter Alkene mit terminaler Kohlenstoff-Kohlenstoff-Doppelbindung unter Erhalt des Kohlenstoffgerüstes, wobei die Kohnlenstoff-Kohlenstoff-Doppelbindung nicht in Konjugation zur funktionellen Gruppe steht, dadurch gekennzeichnet, daß man die funktionalisierten Verbindungen mit endständigen Doppelbindungen in Gegenwart von Rhodium(III)-oder Iridium(III)verbindungen in einer Konzentration von 0.01 bis 10.0 g/kg Reaktionslösung bei Temperaturen von 70 bis 160 °C isomerisiert.

2. Verfahren nach Anspruch 1, dadurch gekennzeichnet, daß man als funktionalisierte Verbindungen mit endständiger Doppelbindung Alpha-Omega-Alkenole, deren Ester, Alpha-Omega-Alkencarbonsäuren oder Alpha-Omega-Alkencarbonsäureester oder Di-(Omega-alkenyl)-Ketone mit unverzweigter Alken-Kohlenstoffkette einsetzt.

3. Verfahren nach Anspruch 1 und 2, dadurch gekennzeichnet, daß man funktionalisierte Verbindungen mit terminaler Kohlenstoff-Kohlenstoff-Doppelbindung einsetzt, bei denen sich zwischen der terminalen Kohlenstoff-Kohlenstoff-Doppelbindung und dem eine funktionelle Gruppe tragenden Kohlenstoff 5 bis 15 Kohlenstoffatome, vorzugsweise 6 bis 10, insbesondere 7 bis 8 Kohlenstoffatome befinden.

4. Verfahren nach einem der Ansprüche 1 bis 3, dadurch gekennzeichnet, daß man als Rhodium(III)verbindungen Rodium(III)-halogenide wie Rhodium(III)chlorid, Rhodium(III)-bromid und/oder Rhodium(III)jodid und/oder andere Rhodium(III)salze wie Rhodium(III)nitrat, Rhodium-(III)sulfat oder deren Hydrate, insbesondere Rhodium(III)chlorid-hydrat einsetzt.

5. Verfahren nach einem der Ansprüche 1 bis 4, dadurch gekennzeichnet, daß man die Isomerisierung bei Temperaturen von 100 bis 130 °C durchführt.

6. Verfahren nach einem der Ansprüche 1 bis 5, dadurch gekennzeichnet, daß man die Isomerisierung in homogener Phase, vorzugsweise in Gegenwart eines Lösemittels oder eines Lösemittelgemisches durchführt.

7. Verfahren nach einem der Ansprüche 1 bis 6, dadurch gekennzeichnet, daß man als Lösemittel einen Alkohol, Ether, Ketone, Amide, Nitrile, Ester, Carbonsäuren, Wasser oder ein Gemisch von ihnen einsetzt.

8. Verfahren nach einem der Ansprüche 1 bis 7, dadurch gekennzeichnet, daß man die Isomerisierung in Gegenwart von 0,1 bis 5 % einer starken Säure, vorzugsweise einer Mineralsäure, bezogen auf die Reaktionslösung, durchführt.

9. Verfahren nach einem der Ansprüche 1 bis 8, dadurch gekennzeichnet, daß man die Isomerisierung unter einem Inertgas, vorzugsweise Stickstoff, oder Luft durchführt.

O.Z. 4179

Abb. 1

Abb. 2

0 262 310

0.Z. 4179

O.Z. 4179

Abb. 3

Abb. 4

PPM

0 262 310

O.Z. 4179

Abb. 5

0 262 310

0.Z. 4179

Abb. 6

Abb. 7

0 262 310

0.Z. 4179

PPM
9  8  7  6  5  4  3  2  1  0

O.Z. 4179

Abb. 8

Abb. 9

0 262 310

0.Z. 4179

8.67
8.82
8.50
8.54
8.09

Abb. 11

8.58

8.75
8.61

7.96
6.76
6.63

Abb. 10

Abb. 12

11,40
11,66

11 8

Abb. 13

10.37          11.09          11,24
                              11,39
11,87                         11,65
12.14

O.Z. 4179

Abb. 14

13.96
14.06

13.85

Abb. 15

13.77

13.99
14.16

Abb. 16

8,32
8.38 8.44
8.54
8.73

10,90

10.85
11.23

11.07

11.89

0.Z. 4179

Abb. 17

21.73

21.96

21.73

Abb. 18

22.12

21.87

22.08

22.36

22.24

22.51

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | FR-A-1 456 900 (INTERNATIONAL FLAVORS AND FRAGRANCES) <br> * Zusammenfassung; Beispiel 8 * <br> --- | 1-9 | C 07 B 35/08 <br> C 07 C 33/025 <br> C 07 C 49/203 <br> C 07 C 57/03 <br> C 07 C 69/52 <br> C 07 C 29/56 <br> C 07 C 45/67 <br> C 07 C 51/353 <br> C 07 C 67/333 |
| A,D | DE-A-2 049 937 (HENKEL) <br> * Ansprüche; Beispiel 18 * <br> ----- | 1-9 | |

RECHERCHIERTE SACHGEBIETE (Int. Cl.4)

C 07 B 35/00
C 07 C 29/00
C 07 C 33/00
C 07 C 45/00
C 07 C 49/00
C 07 C 51/00
C 07 C 57/00
C 07 C 67/00
C 07 C 69/00

Der vorliegende Recherchenbericht wurde für alle Patentansprüche erstellt

| Recherchenort | Abschlußdatum der Recherche | Prüfer |
|---|---|---|
| DEN HAAG | 18-12-1987 | WRIGHT M.W. |

KATEGORIE DER GENANNTEN DOKUMENTE

X : von besonderer Bedeutung allein betrachtet
Y : von besonderer Bedeutung in Verbindung mit einer
  anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
  nach dem Anmeldedatum veröffentlicht worden ist
D : in der Anmeldung angeführtes Dokument
L : aus andern Gründen angeführtes Dokument
&  : Mitglied der gleichen Patentfamilie, übereinstimmendes
  Dokument

EPO FORM 1503 03.82 (P0403)